# EUROPÄISCHE PATENTSCHRIFT

(11) **EP 1 644 054 B1**
(45) Veröffentlichungstag und Bekanntmachung des Hinweises auf die Patenterteilung: **13.06.2007**
(21) Anmeldenummer: 04728167.0
(22) Anmeldetag: 19.04.2004
(51) Int. Cl.: A61L 27/28

(54) **BESCHICHTUNGSSYSTEM FÜR IMPLANTATE ZUR ERHÖHUNG DER GEWEBSVERTRÄGLICHKEIT**
COATING SYSTEM FOR IMPLANTS FOR INCREASING TISSUE COMPATIBILITY
SYSTEME DE REVETEMENT POUR IMPLANTS DESTINE A AUGMENTER LA COMPATIBILITE AVEC DES TISSUS

(30) Priorität: 21.06.2003 DE 10328815
(43) Veröffentlichungstag der Anmeldung: 12.04.2006
(73) Patentinhaber: Biotronik GmbH & Co. KG, 12359 Berlin (DE)
(72) Erfinder: BORCK, Alexander, 91086 Aurachtal (DE); BAYER, Gerd, 91054 Erlangen (DE)
(74) Vertreter: Lindner-Vogt, Karin L.
(86) Internationale Anmeldenummer: PCT/EP2004/004141
(87) Internationale Veröffentlichungsnummer: WO 2004/112857

(56) Entgegenhaltungen:
- EP-A- 0 544 259
- DE-A- 10 135 676
- DENUZIERE A ET AL: "Chitosan-chondroitin sulfate and chitosan-hyaluronate polyelectrolyte complexes: biological properties" BIOMATERIALS, ELSEVIER SCIENCE PUBLISHERS BV., BARKING, GB, Bd. 19, Nr. 14, Juli 1998 (1998-07), Seiten 1275-1285, XP004161388 ISSN: 0142-9612
- LIM S T, MARTIN G P, BERRY D J, BROWN M B: "Preparation and evaluation of the in vitro drug release properties and mucoadhesion of novel microspheres of hyaluronic acid and chitosan" JOURNAL OF CONTROLLED RELEASE, Bd. 66, Nr. 2-3, 2000, Seiten 281-292, XP002291345

## Beschreibung

Die Erfindung betrifft ein Implantat mit einer Beschichtung zur Erhöhung der Gewebsverträglichkeit für Implantate, die einen metallischen Grundkörper besitzen.

Implantate mit einem metallischen Grundkörpern zum dauerhaften oder zumindest mittelfristigen Verbleib im menschlichen oder tierischen Körper führen bekanntermaßen zu Abstoßungsreaktionen im Körper, die die Funktionalität des Implantats und den Heilungserfolg der Therapie mindern. Dieses Problem stellt sich insbesondere bei Stents und Elektroden zur Stimulation von Körpergewebe. Es ist daher bekannt, Beschichtungssysteme auf dem Implantat aufzubringen, die die Gefäßverträglichkeit erhöhen und damit die Gefahr von Abstoßungsreaktionen unterschiedlichster Ausprägung mindern. Teils weisen die metallischen Grundkörper Zwischenschichten auf, die korrosive Prozesse mindern, als auch die Gewebsverträglichkeit verbessern sollen. Ein Beispiel sind Zwischenbeschichtungen aus amorphem Siliziumkarbid.

Polysaccharide sind als biokompatibel bekannt. Typische Vertreter dieser Substanzklasse sind Heparin, Alginat, Chitosan oder Hyaluronsäure. Die letzteren beiden haben sich zum einen als sehr körperverträglich erwiesen, zum anderen sind Beschichtungen aus diesen Komponenten hydrophil und folglich sind die damit versehenen Geräte gut implantierbar.

Mit Polysacchariden im allgemeinen und Hyaluronsäure im speziellen beschichtete Implantate und Verfahren zu deren Beschichtung mit Hyaluronsäure sind aus dem Stand der Technik zahlreich bekannt. So offenbart die US 6,042,876 A einen Führungsdraht für Implantierungszwecke, der mit einem solchen hydrophilen Polysaccharid, wie Hyaluron-säure oder Chondroitinsulfat beschichtet ist.

Die US-A-4,957,744 bezieht sich auf vernetzte Ester von Hyaluronsäure, die für verschiedenste medizinische und kosmetische Artikel sowie pharmazeutische Zusammensetzungen verwendet werden. Die vernetzten Ester resultieren aus der Veresterung von mehrwertigen Alkoholen mit zwei oder mehr Carboxy-Gruppen der Hyaluronsäure. Solche vernetzten Ester sind besonders auf dem Gebiet bioresorbierbarer Kunststoffe für medizinische und chirurgische Artikel verwendbar.

Aus der DE 196 30 563 ist eine implantierbare Stimulationselektrode bekannt, die eine erhöhte Gewebsverträglichkeit zeigt. Dies wird dadurch erreicht, dass eine im wesentlichen die gesamte äußere Oberfläche der Stimulationselektrode bildende, dünne, spezifisch funktionalisierte organische Beschichtung vorgesehen ist, die auf Grund irreversibler Physisorption oder kovalenter Bindung an der darunter liegenden Oberfläche fest haftet. Als Beschichtungsmaterialien werden u.a. Silane und synthetische Polymere wie Polystyrensulfonat, Polyvinylsulfonat oder Polyallylamin vorgeschlagen. Die organische Beschichtung kann auch mehrschichtig sein, wobei an der äußeren Oberfläche insbesondere Polyethylenoxid oder Polyethylenglycol terminiert ist. Ferner wird angesprochen, dass die organische Schicht einen medizinischen Wirkstoff, insbesondere ein entzündungshemmendes Medikament beinhaltet, der aus der organischen Beschichtung diffusions- oder lösungsgesteuert austragbar ist.

Die geschilderten Verbesserungen durch die Beschichtung der Stimulationselektrode führen zwar bereits zu einer deutlichen Minderung der temporären Reizschwellenerhöhung, sind aber relativ aufwendig und damit kostspielig in der Umsetzung und fordern wegen der synthetischen Natur der verwendeten Materialien umfangreiche Tests zur Evaluierung der Biokompatibilität. Weiterhin ist es im Falle des gewünschten Zusatzes von entzündungshemmenden Wirkstoffen notwendig, die Materialeigenschaften der Wirkstoffe und der sie einbettenden organischen Beschichtung durch umfangreiche Tests aufeinander abzustimmen.

Schließlich bezieht sich die WO 8802623 A1 auf Biomaterialien mit biokompatibler Oberfläche, wobei unter einer Vielzahl von Ausgangsmaterialien und Bindungsmechanismen unter anderem die Verwendung von Hyaluronsäure zur Herstellung einer biokompatiblen Kontaktlinse offenbart wird.

Sofern die vorgenannten Druckschriften Beschichtungssysteme für medizinische Gerätschaften und insbesondere Stents und Stimulationselektroden betreffen, weisen diese den Nachteil auf, dass die erzielten Beschichtungen keine ausreichenden Haftfestigkeiten auf der Substratoberfläche erzielen, die Beschichtungen die feinen Strukturen der Implantate nur ungleichmäßig bedecken und deren Aufbringung technisch sehr aufwendig ist.

Aufgabe der vorliegenden Erfindung ist es, ein Implantat mit einer Beschichtung zur Verbesserung der Gewebsverträglichkeit bereitzustellen, das die vorgenannten Nachteile des Standes der Technik überwindet. Die Beschichtung des Implantats soll insbesondere eine sehr hohe Biokompatibilität besitzen und zudem von sich aus entzündungshemmend wirken. Ferner soll die Beschichtung aus möglichst wenig und leicht verarbeitbaren Komponenten bestehen, so dass die Herstellung vereinfacht wird.

Diese Aufgabe wird durch das erfindungsgemäße Implantat mit einer Beschichtung nach Anspruch 1 gelöst.

Die erfindungsgemäßen Implantate weisen eine durch Physisorption und/oder kovalente Bindung gebundene Beschichtung auf. Die Beschichtung bedeckt den metallischen Grundkörper und gegebenenfalls eine oder mehrere auf dem Grundkörper aufgebrachte Zwischenschichten. Zur Erhöhung der Gewebsverträglichkeit ist auf dem Implantat eine Beschichtung aus
(a) Chitosan und
(b) Hyaluronsäure und/oder Hyaluronsäure-Derivaten
aufgebracht.

Es hat sich überraschenderweise gezeigt, dass die Aufbringung einer solchen Polysaccharidschicht zu einer wesentlichen Verbesserung der Gewebsverträglichkeit führt. Ferner zeichnen sich Hyaluronsäure, seine Derivate und Chitosan durch ihre sehr gute Biokompatibilität aus, da die Materialien natürlichen Ursprungs sind. Weiterhin hat es sich gezeigt, dass zumindest Hyaluronsäure als auch seine Derivate eine eigenständige entzündungshemmende Wirkung besitzen und damit wirkungsvoll Gewebsirritationen verhindert oder zumindest stark vermindert werden können.

Hyaluronsäure (Hyaluronan) ist ein einfaches Glykosaminoglykan der extrazellulären Matrix. Es wird an der Oberfläche von Fibroblasten synthetisiert und kommt als einziges Glykosaminoglykan nicht als Proteoglykan vor. Hyaluronsäure ist eine hochmolekulare Verbindung mit M_{R} zwischen 50.000 und mehreren Millionen. Grundbaustein der Hyaluronsäure ist ein aus D-Glucuronsäure und N-Acetyl-d-glucosamin in β1-3-glykosidischer Bindung aufgebautes Aminodisaccharid, das mit der nächsten Einheit β1-4-glykosidisch verbunden ist:

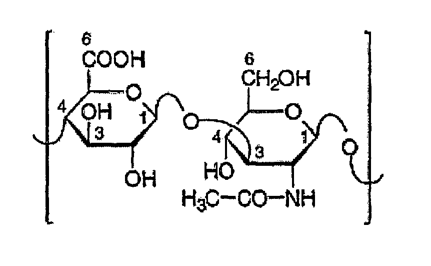

Die unverzweigte Kette der Hyaluronsäure besteht aus 2.000-10.000 solcher Einheiten. Durch Hyaluronidasen werden β-glykosidische Bindungen hydrolysiert und so die Hyaluronsäure zu kleineren Bruchstücken abgebaut. Die - meist als Kalium-Salz - im Handel befindliche Hyaluronsäure ist aus menschlichen Nabelschnüren oder Hahnenkämmen isoliert, wird aber zunehmend biotechnologisch durch bakterielle Fermentation hergestellt.

Zur Modifizierung von Hyaluronsäure, d.h. Darstellung von Hyaluronsäure-Derivaten, werden literaturbekannte Verfahren eingesetzt (z. B. Danishefsky, Arch. Biochem. Biophys., 90, 1960, S. 114 ff.; Nagasawa, Carbohydr. Res., 58, 1977, S. 47 ff.; Ayotte, Carbohydr. Res. 145, 1986, S. 267 ff.; Ogamo, Carbohydr. Res. 193, 1989, S. 165 ff.; Jesaja, Can. J. Chem.; 67, 1989, S. 1449 ff.; Mulloy, Carbohydr. Res. 255, 1994, S. 1 ff.). Dabei handelt es sich um regio- und stereoselektive und nicht regio- und stereoselektive (statische) Reaktionen. Basierend auf diesem Verfahren kann Hyaluronsäure insbesondere durch N- und O-Desulfatierung, O-Desulfatierung, 6-O-Desulfatierung, Deacetylierung oder Acetylierung sowie Sulfatierung, Acylierung mit aliphatischen oder aromatischem Rest verändert werden. Insbesondere können durch die bekannten Verfahren Aminogruppen, Sulfat- oder Carboxylreste unter Anwendung von Schutzgruppenchemie und bekannten, zum Teil regioselektiven Reaktionen der organischen Chemie eingeführt werden.

Unter dem Begriff "Hyaluronsäue-Derivate" im Sinne der Erfindung werden demnach alle durch gezielte Modifizierungen der natürlichen Hyaluronsäure strukturell zum Ausgangsprodukt veränderten Reaktionsprodukte verstanden. Unter dem Begriff "Hyaluronsäure und Hyaluronsäure-Derivate" werden ferner alle polyelektrolytischen Salze derselben, z.B. Natrium-, Kalium-, Magnesium- und Kalziumsalze, verstanden. Als "Modifizierungen" im erfindungsgemäßen Sinne werden die aufgeführten und weiteren bekannten Reaktion der organischen Chemie zur Umsetzung der funktionellen Gruppen der Hyaluronsäure angesehen.

Hyaluronsäure, die Hyaluronsäure-Derivate und Chitosan können als Einzelsubstanzen, Co- oder Blockpolymere aus Hyaluronsäure, Hyaluronsäure-Derivaten und Chitosan, als auch in Form von Mischungen der vorgenanten Einzelsubstanzen und Polymere kovalent und/oder durch Physisorption auf dem Implantat immobilisiert werden.

Eine kovalente Anbindung der Polysaccharidschicht an die Oberfläche des Implantats erfolgt vorzugsweise durch Einpunkts- oder Mehrpunktsaufhängung an Spacer. Weiterhin wird vorzugsweise durch Vernetzung einer zuvor aufgebrachten (primären) Polysaccharidschicht eine mechanische und/oder chemische Stabilisierung des Beschichtungsmaterials gegen enzymatischen und hydrolytischen Abbau als auch gegen mechanischen Stress erreicht. Die Immobilisierung der Polysaccharidschicht auf der Oberfläche der Implantate kann nach bekannten Methoden der Immobilisierung von Enzymen, Methoden der Membranherstellung, Kunststoffverarbeitung, Polymerchemie, der Peptid-, Protein- und Zuckerchemie über kovalente Bindungen mit und ohne Verwendung von Spacern, mittels Einpunkts- und Mehrpunktaufhängung, Endpunktaufhängung als Mono- oder Multilayer oder mit zusätzlicher Stabilisierung durch Quervernetzung erfolgen.

Als vorteilhaft hat sich eine Beschichtung mit einer Schichtdicke im Bereich zwischen 10-400 µm, insbesondere 50-120 µm, erwiesen. Bei den genannten Schichtdicken konnte noch kein signifikanter Effekt auf die Funktionalität der Implantate festgestellt werden.

Weiterhin ist bevorzugt, wenn die Hyaluronsäure oder die Hyaluronsäure-Derivate nach Sterilisation noch ein durchschnittliches Molekulargewicht im Bereich von ca. 300.000-500.000, insbesondere 380.000-420.000 g/mol aufweisen. Im beanspruchten Molekulargewichtsbereich erreicht die eigenständige therapeutische Wirkung der Hyaluronsäure und seiner Derivate ein Maximum (Papakonstantinou, G. Karakiulakis, O. Eickelberg, A.P. Perruchoud, L.H. Block, and M. Roth ; A 340 kDa hyaluronic acid secreted by human vascular smooth muscle cells regulates their proliferation and migration, Glycobiology 1998, 8, 821-830).

Ein weiterer vorteilhafter Aspekt der erfindungsgemäßen Lehre liegt in der gezielten Beeinflussung des in vivo Degradationsverhalten des Biopolymers. Unter dem Begriff "Degradationsverhalten" wird der durch chemische, thermische, oxidative, mechanische oder biologische Prozesse stattfindende Abbau der erfindungsgemäßen Polysaccharidschicht im lebendem Organismus über die Zeit verstanden. Einerseits soll sichergestellt werden, dass zumindest in den ersten Wochen nach der Implantation lokale Entzündungserscheinungen des anliegenden Gewebes gelindert oder gar vermieden werden. Andererseits soll die Beschichtung über einen bestimmten Zeitraum eine Oberflächenadsorption von hochmolekularen Biomolekülen auf dem Implantat verhindern oder zumindest deutlich zurückdrängen.

Vorzugsweise ist die Polysaccharidschicht derart beschaffen ist, dass die in vivo Degradation der Polysaccharidschicht von außen in Richtung des Grundkörpers des Implantats verlangsamt ist. Das Degradationsverhalten kann dabei kontinuierlich oder sprunghaft verändert werden. Nach letzterer Variante umfasst die Polysaccharidschicht zumindest zwei Teilschichten mit unterschiedlichem Degradationsverhalten, wobei das Degradationsverhalten innerhalb jeder Teilschicht kontinuierlich veränderlich oder konstant über die Teilschicht festlegbar ist. Die Herstellung derartiger Beschichtungen kann mit Hilfe an sich bekannter Sprüh- und Tauchbeschichtungsverfahren erfolgen.

Vorzugsweise ist die Polysaccharidschicht derart beschaffen, dass ein dem Grundkörper des Implantats abgewandter, äußerer Bereich der Polysaccharidschicht innerhalb von 100 Tagen in vivo abgebaut wird. Der äußere Bereich ist vorzugsweise 10 bis 250 µm, insbesondere 50 bis 150 µm, dick. Wenn die Polysaccharidschicht aus zumindest zwei Teilschichten mit unterschiedlichem Degradationsverhalten besteht, ist zur Erreichung dieses Ziels eine äußere Teilschicht derart modifiziert, dass sich diese äußere Teilschicht um mehr als 50 Gew% innerhalb von 100 Tagen in vivo abbaut. Die äußere Teilschicht ist vorzugsweise 10 bis 250 µm, insbesondere 50 bis 150 µm, dick.

Es hat sich ferner überraschenderweise gezeigt, dass in Gegenwart der erfindungsgemäßen Polysaccharidschicht auch die Oberflächenadsorption von hochmolekularen Biomolekülen auf dem Implantat verhindert oder zumindest deutlich zurückgedrängt ist. Vorzugsweise ist daher die Polysaccharidschicht derart beschaffen, dass ein dem Grundkörper des Implantats zugewandter, innerer Bereich der Polysaccharidschicht zumindest nicht vollständig innerhalb von zwei Jahren in vivo abgebaut wird. Der innere Bereich ist vorzugsweise 3 bis 50 µm, insbesondere 5 bis 20 µm, dick. Wenn die Polysaccharidschicht aus zumindest zwei Teilschichten mit unterschiedlichem Degradationsverhalten besteht, ist zur Erreichung dieses Ziels insbesondere eine innere Teilschicht, die sich unmittelbar der darunter liegenden Oberfläche des Grundkörpers oder gegebenenfalls einer hierauf aufgebrachten Zwischenschicht anschließt, derart modifiziert, dass sich diese innere Teilschicht um nicht mehr als 20 Gew% innerhalb von zwei Jahren in vivo abbaut. Die äußere Teilschicht ist vorzugsweise 3 bis 50 µm, insbesondere 5 bis 20 µm, dick.

Zur Beeinflussung des Degradationsverhaltens kann das Degradationsverhalten von Hyaluronsäure und seiner Derivate u.a. durch Quervernetzung beeinflusst werden. Hierzu wird generell auf die in der Literatur zahlreichen beschriebenen Verfahren zur Durchführung der einzelnen Vernetzungsreaktionen und ausdrücklich auf den Gegenstand der US 4,582,865, US 5,550,187, US 5,510,121 und WO 00/46252 verwiesen. So kann eine Quervernetzung z. B. mit Hilfe der folgenden Reagenzien durchgeführt werden:

Formaldyhyd, Glutaraldehyd, Divinylsulfon, Polyanhydride, Polyaldehyde, Carbodiimide, Epichlorohydrin, Ethylenglykoldiglycidylether, Butandiol-diglycidylether, Polyglycerol-polyglycidylether, Polyethylenglykol- diglycidylether, Polypropylenglykol-diglycidylether oder bis- oder Polyepoxy-Vernetzer, wie 1,2,3,4-Diepoxybutan oder 1,2,7,8-Diepoxyoctan.

Der Zusammenhang zwischen Vernetzungsgrad und Degradationsverhalten kann über herkömmliche Testverfahren ermittelt werden. Ein unterschiedlicher Vernetzungsgrad führt zu einem unterschiedlichen Quellverhalten der Polysaccharidschicht. Das Quellverhalten lässt sich u.a. gravimetrisch bestimmen. Weiterhin lässt sich der Vernetzungsgrad auch durch infrarotspektroskopische Analyse an vernetzten Hyaluronsäurefolien bestimmen. Der Bezug zur Degradation kann durch eine GPC Analytik, d.h. durch Molmassenbestimmung degradierter Hyaluronsäure, an Eluenten hergestellt werden.

Der Einfluss der genannten Modifikationen auf das in vivo Degradationsverhalten ist allgemein bekannt. Da das Abbauverhalten aber u.a. auch von weiteren geometrischen und physiologischen Faktoren abhängt, ist in der Regel eine individuelle Anpassung des Systems an die jeweiligen Erfordernisse notwendig.

Die Beschichtung kann in der Regel auf alle bekannten metallischen Implantate aufgebracht werden. Die dünne Polysaccharidschicht aus Hyaluronsäure und/oder Hyaluronsäure-Derivaten sowie Chitosan wird dazu mittels gängiger Sprühverfahren oder aus der Lösung abgeschieden.

Die prinzipielle Herstellung einer kovalent anhaftenden Polysaccharidschicht wird in der WO 00/56377 beschrieben, deren Offenbarung vollumfänglich mit einbezogen wird. Eine Substratoberfläche wird dazu mit reaktiven Funktionalitäten modifiziert, aktivierte Hyaluronsäure wird bereit gestellt und diese wird dann unter geeigneten Bedingungen kovalent an die reaktiven Funktionalitäten gebunden. In eben gleicher Weise lässt sich die erfindungsgemäße Polysaccharidschicht an die Oberfläche der Implantate binden.

Weiterhin offenbart die DE 196 30 563 ein Verfahren zur Verbesserung der Haftung einer Beschichtung infolge verstärkter Physisorption bzw. kovalenter Bindung. In einem ersten Schritt wird eine reaktive Funktionalität auf der Substratoberfläche erzeugt. Die reaktive Funktionalität umfasst insbesondere Amine, Aldehyde, Sulfide, Alkohole, Säurehalogenide und Isocyanate. An die genannte Funktionalität kann dann - unter Rückgriff auf an sich bekannte Kopplungsverfahren - die erfindungsgemäße Polysaccharidschicht kovalent gebunden werden.

Das erfindungsgemäße Beschichtungssystem kann durch Einbettung therapeutischer Wirkstoffe, die durch den allmählichen Abbau der Beschichtung bzw. Diffusion in das umliegende Gewebe freigesetzt werden, ergänzt werden.

Weiterhin ist bevorzugt, wenn die Polysaccharidschicht eine Haftvermittlerschicht aus Chitosan umfasst. Die Haftvermittlerschicht schließt sich unmittelbar dem Grundkörper und ggf. der darauf aufgebrachten Zwischenschicht an. Es hat sich überraschenderweise gezeigt, dass in Gegenwart einer solchen Haftvermittlerschicht sehr gleichmäßige und stark haftende Beschichtungen erzeugt werden können. Zudem ist Chitosan Werkstoff natürlichem Ursprungs und damit gut bioverträglich. Die als Haftvermittlerschicht ist vorzugsweise 0,1 bis 50 µm, insbesondere 1 bis 10 µm, dick und kann ebenso wie die Hyaluronsäure und ihre Derivate zur Beeinflussung Ihres Degradationsverhaltens modifiziert werden. Insbesondere kann die Haftvermittlerschicht derart ausgebildet sein, dass sie als innere Teilschicht oder innerer Bereich der Polysaccharidschicht im oben genannten Sinne agieren kann.

Nach einer weiteren bevorzugten Variante der Erfindung beinhaltet die Polysaccharidschicht zumindest in Teilbereichen oder Teilschichten Chitosan. Hierdurch kann das Haftvermögen der Polysacharidschicht weiter verbessert werden und es können auch auf den oft sehr komplexen Geometrien der Substrate gleichmäßige Beschichtungen erzeugt werden.

Die Stabilität der Polysacharidschicht kann gesteigert werden, wenn durch Quarternisierung der aminischen Funktionen des Chitosans polykationische Ladungen erzeugt werden. Werden Hyaluronsäure und/oder seine Derivate als polyanionische Präperate zugemengt, so bildet sich Symplexgele. Die schon sehr starke Ion/Ion-Wechselwirkung zwischen den Komponenten kann durch Quervernetzung weiter erhöht werden. Ein Gewichtsanteil des Chitosans am Gesamtgewicht der Polysaccharidschicht beträgt vorzugsweise nicht mehr als 50%.

In den ersten Wochen nach der Implantation von Stimulationselektroden ist allgemein eine temporäre Reizschwellenerhöhung festzustellen, die sich auf lokale Entzündungserscheinungen des anliegenden Gewebes zurückführen lassen. Diese Entzündungserscheinungen führen außerdem zu einem ungünstigen Einwachsverhalten der Stimulationselektroden, was langfristig die Stimulationseigenschaften des Systems negativ beeinflusst. Durch das erfindungsgemäße Beschichtungssystem kann dieses Problem behoben werden. Daher wird die Verwendung des Beschichtungssystems in diesem Zusammenhang beansprucht.

Im Zuge einer akuten Myokardtherapie werden sehr häufig Stents implantiert. Durch spezifische mikrobiologische Prozesse kommt es jedoch oftmals im Laufe der Zeit zu einem erneuten Verschluss des geöffneten Gefäßes (Restenose). Dem kann wirkungsvoll mit dem erfindungsgemäßem Beschichtungssystem entgegengewirkt werden. Daher wird die Verwendung des Beschichtungssystems in diesem Zusammenhang beansprucht.

Nachfolgend wird die Erfindung anhand von Ausführungsbeispielen näher erläutert.

### Ausführungsbeispiel 1 - Chitosan als Teilschicht

Die nachfolgenden Verfahrensbeschreibungen eignen sich insbesondere zur Herstellung der erfindungsgemäßen Beschichtung auf Stents oder Stimulationselektroden.

Die Implantatsoberfläche wurde vorgereinigt, entfettet und unter leichtem Rühren für 10 Minuten bei Raumtemperatur in eine 0,5 bis 2%ige Essigsäure mit einer Chitosankonzentration zwischen 0,1% und 0,5% gerührt. Das Molekulargewicht des Chitosans betrug zwischen 100.000 g/mol und 1.000.000 g/mol. Anschließend wurde das Implantat entnommen und getrocknet.

Alternativ konnte eine dünne Schicht aus Chitosan durch Aufsprühen auf das Implantat aufgebracht werden. Hierzu wurde eine 0,5%ige Chitosanlösung in einer 0,5%igen Essigsäure angesetzt. Die vorgereinigten Implantate wurden 5 bis 20 mal im Abstand von 15 bis 30 Sekunden für 0,5 bis 1,0 sec mit Hilfe einer Airbrushpistole besprüht, wobei zwischen den Sprühschritten die Implantate bei 40°C bis 70°C getrocknet wurden. Die aufgebrachten Schichten wiesen eine Schichtdicke von 1 µm bis 10µm auf.

Nach Trocknung wurde die Implantate unter leichtem Rühren für 10 Minuten bei Raumtemperatur in eine wässrige Lösung von Hyaluronsäure mit einem Molekulargewicht von mindestens 1.000.000 g/mol gelegt. Nach Entnahme und Trocknung wurde die Implantate für mindestens 2 h bei ca. 30°C bis 40°C in eine Vernetzerlösung von 2 bis 4 ml Glutaraldehyd in einem Wasser-Aceton Gemisch getaucht. Danach wurde die Vernetzerlösung ausgetauscht und die Vernetzung 2 h fortgeführt. Die Versuchsbedingungen führen auch zu einer Vernetzung von Chitosan mit Glutaraldehyds. Die säurekatalysierte Reaktion des Aldehyds mit dem Amin des Chitosans findet unter Bildung einer Schiffschen Base statt.

Anschließend wurde die Implantate mehrfach mit destilliertem Wasser gespült und mit einer verdünnten Lösung von Natriumcyanoborhydrid reduktiv fixiert sowie mehrfach mit deionisiertem Wasser gespült. Die Nachbehandlung führt zur Reduktion der Schiffschen Base und freier Aldehydfunktionen. Nach Entnahme wurde die Probe für 24 Stunden bei 50°C im Trockenschrank getrocknet.

Das Chitosan fungiert als Haftvermittler, da Chitosan selbst im neutralen Bereich (Blut) schwer löslich ist. Zudem liegt das Chitosan vernetzt vor und bildet durch die Vernetzung mit Hilfe des Glutaraldehyd auch einen kovalenten Verbund zur aufgebrachten Hyaluronsäureschicht. Die dünne Haftvermittlerschicht aus Chitosan von 0,1 µm bis 50 µm, vorzugsweise von 1 µm bis 10 µm, hat keine signifikante Beeinträchtigung der elektrischen Übertragungseigenschaften der Elektrode zur Folge.

### Ausführungsbeispiel 2 - Chitosan als Zusatz

Neben den Polyanionen Hyaluronsäure bzw. seinen Hyaluronsäure-Derivaten enthält die Beschichtung noch das polykationische Chitosan. Durch das Amin des Chitosans liegt eine weitere funktionelle Gruppe für den Vernetzer Glutardialdehyd vor. Die Aldehydfunktion kann sowohl mit der Aminfunktion des Chitosans als auch mit der Carbonyl- bzw. Hydroxylfunktion der Hyaluronsäure reagieren. Durch diese Reaktionen kann der Vernetzungsgrad zusätzlich erhöht und die ionische Wechselwirkung zwischen den Polyanionen und Polykationen zusätzlich verstärkt werden. Das Schichtsystem aus Polyanionen und Polykationen kann durch abwechselndes Besprühen der Implantate mit Lösungen gewünschter Konzentrationen von Chitosan, Hyaluronsäure und Hyaluronsäure-Derivaten hergestellt werden.

Hierbei werden vorgereinigte Implantate abwechselnd mit einer wässrigen Lösung aus Hyaluronsäure oder Hyaluronsäure-Derivat und in Essigsäure gelöstem Chitosan besprüht. Dabei beträgt die Konzentration der Hyaluronsäure oder Hyaluronsäure-Derivate 0,1% bis 1%, vorzugsweise 0,2% bis 0,5%. Die Konzentration der Essigsäure beträgt 0,1 % bis 2%, vorzugsweise 0,5% bis 1 %. Die Konzentration des Chitosans beträgt 0,1% bis 1%, vorzugsweise 0,2% bis 0,5%. Das Molekulargewicht der Hyaluronsäure oder der Hyaluronsäure-Derivate beträgt mindestens 1.000.000 g/mol und das Molekulargewicht des Chitosans mindestens 100.000 g/mol. Beide Lösungen werden im Abstand von 2 Sekunden bis 60 Sekunden, vorzugsweise 15 Sekunden bis 30 Sekunden, mit Hilfe eines Sprühverfahrens abwechselnd auf die Implantate aufgebracht. Durch die Wahl der Konzentration an Hyaluronsäure bzw. Chitosan und der jeweiligen Sprühdauer kann der jeweilige Anteil an Polyanionen und Polykationen eingestellt werden. Der Gewichtsanteil an Chitosan am gesamten Schichtsystem beträgt nicht mehr als 50%. Die Anzahl der Sprühschritte bestimmt die Schichtdicke des gesamten Schichtsystems. So werden bei 60 Sprühschritten mit einer Sprühdauer von 0,5 Sekunden mit üblichen Airbrushpistolen Schichtdicken zwischen 5 µm und 10 µm, gemessen im trockenen Zustand, erreicht. Nach der Beschichtung wird das Implantat getrocknet und anschließend für mindestens 2 h bei ca. 30°C bis 40°C in eine Vernetzerlösung von 2 bis 4 ml Glutaraldehyd in einem Wasser-Aceton Gemisch getaucht. Danach wird die Vernetzerlösung für mindestens weitere 2 h ausgetauscht. Anschließend wird das Implantat mehrfach mit destilliertem Wasser gespült und mit einer verdünnten Lösung von Natriumcyanoborhydrid reduktiv fixiert, sowie mehrfach mit deionisiertem Wasser gespült. Nach Entnahme wird die Probe für 24 Stunden bei 50°C im Trockenschrank getrocknet.

### Untersuchungen zum Quellverhalten

Ein unterschiedlicher Vernetzungsgrad führt zu einem unterschiedlichen Quellverhalten der Polysaccharidschicht. Das Quellverhalten lässt sich u.a. gravimetrisch bestimmen. Weiterhin lässt sich der Vernetzungsgrad auch durch infrarotspektroskopische Analyse an vernetzten Hyaluronsäurefolien bestimmen. Der Bezug zur Degradation kann durch eine GPC Analytik, d.h. durch Molmassenbestimmung degradierter Hyaluronsäure, an Eluenten hergestellt werden.

Um den Einfluss von Vernetzungsparametern auf die Vernetzung und damit auch auf das Quellverhalten zu bestimmen, wurden die Parameter Temperatur, Wassergehalt, Art des Vernetzers und die Vernetzungsdauer variiert. Zur Bestimmung der Korrelation zwischen Quellverhalten und den Vernetzungsparametern wurden Hyaluronsäurefolien gegossen und vernetzt.

### Beispiele 1 bis 8 - Versuche zum Quellverhalten

Das Verfahren nach Beispiel 1 gliederte sich in folgende Schritte:
(a) Ansetzen einer 1 %igen Hyaluronsäurelösung;
(b) Ausgießen von 3 ml 1%iger Hyaluronsäurelösung in Petrischalen mit 4 cm Durchmesser und anschließendes Trocknen;
(c) Zugabe von 4 ml Vernetzerlösung zu den Folien bei Raumtemperatur (20°C), wobei die Vernetzerlösung aus 240 ml Aceton, 80 ml 25%ige Glutaraldehydlösung und 1,6 ml 3 molare Salzsäure bestand;
(d) Vernetzungsdauer 20 Stunden, wobei die Vernetzerlösung nach 4 Stunden ausgetauscht wurde;
(e) Entnahme und Spülen mit deionisiertem Wasser;
(f) Zugabe von 4 ml 2,2 %ige NaBH₃CN-Lösung;
(g) Spülen mit deionisiertem Wasser;
(h) Trocknen.

Die weiteren Beispiele 2 bis 8 wichen bei sonst gleicher Verfahrensführung wie folgt ab:
In Beispiel 2 betrug die Vernetzungsdauer im Schritt (d) 4 h ohne Wechsel der Vernetzerlösung.
In Beispiel 3 betrug die Vernetzungsdauer im Schritt (d) 2 h ohne Wechsel der Vernetzerlösung.
In Beispiel 4 enthielt die in Schritt (c) genannte Vernetzerlösung zusätzlich 20 ml deionisiertes Wasser.
In Beispiel 5 enthielt die in Schritt (c) genannte Vernetzerlösung zusätzlich 100 ml deionisiertes Wasser.
In Beispiel 6 enthielt die in Schritt (c) genannte Vernetzerlösung 80 ml 25%ige Formaldehydlösung anstelle der Glutaraldehydlösung.
In Beispiel 7 wurde die Vernetzung in Schritt (d) bei 30°C durchgeführt und die Vernetzungsdauer im Schritt (d) betrug 6,5 h, wobei nach 1,5 h die Vernetzerlösung ausgetauscht wurde.
In Beispiel 8 wurde die Vernetzung in Schritt (d) bei 30°C durchgeführt und die Vernetzungsdauer im Schritt (d) betrug 7 h, wobei nach 2 h die Vernetzerlösung ausgetauscht wurde.

Nach Trocknen der vernetzten Folien wurden diese gewogen und anschließend in deionisiertem Wasser für 30 Minuten gespült, kurz abgetupft und erneut gewogen, um das Quellverhalten, welches mit dem Vernetzungsgrad korreliert, zu bestimmen.

Die ermittelten Quellfaktoren lassen sich der nachfolgenden Tabelle entnehmen:

**Tabelle 1 - Quellfaktoren**

| | | | | | | | | |
|---|---|---|---|---|---|---|---|---|
| Beispiel | 1 | 2 | 3 | 4 | 5 | 6 | 7 | 8 |
| Quellfaktor | 6 | 14 | 75 | 7 | 7 | 34 | 10 | 13 |

Die beispielhaft aufgeführten Versuche zur Vernetzung lassen folgende Schlussfolgerungen zu:

Die Vernetzungsdauer hat einen signifikanten Einfluss auf den Vernetzungsgrad, was sich im Quellverhalten nieder schlägt. Bei einer Vernetzungsdauer von nur 2 Stunden werden Hyaluronsäurefolien erhalten, welche instabil sind und sich innerhalb weniger Stunden in Wasser auflösen. Hingegen werden bei einer Vernetzungsdauer von 4 Stunden stabile Hyaluronsäurefolien erhalten, welche aber gegenüber den Folien des Standardverfahrens einen höheren Quellfaktor zeigen. Der Wassergehalt der Vernetzerlösung hat im untersuchten Bereich keinen starken Einfluss auf den Quellfaktor und somit den Vernetzungsgrad. Die Verwendung von Formaldehyd anstelle von Glutaraldehyd führt zu vernetzten Hyaluronsäurefolien mit einem wesentlich höheren Quellfaktor. Dies lässt sich vermutlich auf die kürzere Kettenlänge des Formaldehyds zurückführen. Der kürzere Vernetzer Formaldehyd führt somit zu leicht vernetzten Hyaluronsäurefolien. Eine Vernetzung bei einer Temperatur von 30°C und einer Vernetzungsdauer von 7 h führt zu Hyaluronsäurefolien mit einem etwas höheren Quellfaktor und somit geringeren Vernetzungsgrad.

## Patentansprüche

1. Implantat mit einem metallischen Grundkörper, der gegebenenfalls mit einer oder mehreren Zwischenschichten bedeckt ist, **dadurch gekennzeichnet, dass** auf dem Implantat zur Erhöhung der Gewebsverträglichkeit eine Beschichtung aus einer Polysaccharid schicht aus
(a) Chitosan und
(b) Hyaluronsäure und/oder Hyaluronsäure-Derivaten
aufgebracht ist.

2. Implantat nach Anspruch 1, **dadurch gekennzeichnet, dass** die Polysaccharidschicht in Teilbereichen oder Teilschichten Chitosan beinhaltet.

3. Implantat nach Anspruch 2, **dadurch gekennzeichnet, dass** die Polysaccharidschicht eine Haftvermittlerschicht aus Chitosan umfasst.

4. Implantat nach Anspruch 3, **dadurch gekennzeichnet, dass** die Haftvermittlerschicht 0,1 bis 50 µm, insbesondere 1 bis 10 µm, dick ist.

5. Implantat nach einem der vorhergehenden Ansprüche, **dadurch gekennzeichnet, dass** ein Anteil des Chitosans am Gesamtgewicht der Polysaccharidschicht nicht mehr als 50 Gew.% beträgt.

6. Implantat nach Anspruch 1, **dadurch gekennzeichnet, dass** die Hyaluronsäure und Hyaluronsäure-Derivate nach einer Sterilisation des Implantates ein durchschnittliches Molekulargewicht zwischen 300.000 bis 500.000 Dalton aufweisen.

7. Beschichtungssystem nach Anspruch 6, **dadurch gekennzeichnet, dass** das durchschnittliche Molekulargewicht zwischen 380.000 bis 420.000 Dalton liegt.

8. Implantat nach einem der vorhergehenden Ansprüche, **dadurch gekennzeichnet, dass** die Polysaccharidschicht derart beschaffen ist, dass die in vivo Degradation der Polysaccharidschicht von außen in Richtung des Grundkörpers des Implantates verlangsamt ist.

9. Implantat nach Anspruch 8, **dadurch gekennzeichnet, dass** ein innerer Bereich der Polysaccharidschicht zumindest nicht vollständig innerhalb von zwei Jahren in vivo abbaubar ist.

10. Implantat nach Anspruch 9, **dadurch gekennzeichnet, dass** der innere Bereich 3 bis 50 µm, insbesondere 5 bis 20 µm, dick ist.

11. Implantat nach Anspruch 8, **dadurch gekennzeichnet, dass** ein äußerer Bereich der Polysaccharidschicht innerhalb von 100 Tagen in vivo abbaubar ist.

12. Implantat nach Anspruch 11, **dadurch gekennzeichnet, dass** der äußere Bereich 10 bis 250 µm, insbesondere 50 bis 150 µm, dick ist.

13. Implantat nach Anspruch 8, **dadurch gekennzeichnet, dass** die Polysaccharidschicht zumindest zwei Teilschichten mit unterschiedlichem Degradationsverhalten umfasst, wobei das Degradationsverhalten innerhalb jeder Teilschicht kontinuierlich veränderlich oder konstant über die Teilschicht festlegbar ist.

14. Implantat nach Anspruch 13, **dadurch gekennzeichnet, dass** die Polysaccharidschicht eine innere Teilschicht umfasst, die um nicht mehr als 20 Gew.% innerhalb von 2 Jahren in vivo abbaubar ist.

15. Implantat nach Anspruch 14, **dadurch gekennzeichnet, dass** der innere Teilschicht 3 bis 50 µm, insbesondere 5 bis 20 µm, dick ist.

16. Implantat nach Anspruch 13, **dadurch gekennzeichnet, dass** die Polysaccharidschicht eine äußere Teilschicht umfasst, die zumindest um mehr als 50 Gew.% innerhalb von 100 Tagen in vivo abbaubar ist.

17. Implantat nach Anspruch 16, **dadurch gekennzeichnet, dass** der äußere Teilschicht 10 bis 250 µm, insbesondere 50 bis 150 µm, dick ist.

18. Implantat nach einem der vorhergehenden Ansprüche, **dadurch gekennzeichnet, dass** eine Schichtdicke der Polysaccharidschicht zwischen 10-400 µm liegt.

19. Implantat nach Anspruch 18, **dadurch gekennzeichnet, dass** die Schichtdicke 50-120 µm beträgt.

20. Implantat nach einem der vorhergehenden Ansprüche, **dadurch gekennzeichnet, dass** die Hyaluronsäure, die Hyaluronsäure-Derivate und das Chitosan als Einzelsubstanzen, Co- oder Blockpolymere aus Hyaluronsäure, Hyaluronsäure-Derivaten und Chitosan oder in Form von Mischungen der vorgenannten Einzelsubstanzen und Polymere Bestandteil der Polysaccharidschicht sind.

21. Implantat nach einem der vorhergehenden Ansprüche, **dadurch gekennzeichnet, dass** die Polysaccharidschicht kovalent oder durch Physisorption auf dem Implantat immobilisiert ist.

22. Implantat nach einem der vorhergehenden Ansprüche, **dadurch gekennzeichnet, dass** das Implantat ein endovaskuläres Implantat, insbesondere ein Stent ist.

23. Implantat nach einem der Ansprüche 1 bis 21, **dadurch gekennzeichnet, dass** das Implantat eine Stimulationselektrode zur Verwendung mit einem implantierbaren Gewebsstimulator, insbesondere einem Herzschrittmacher, Defibrillator, Knochen- oder Neurostimulator, ist.

## Claims

1. An Implant having a metallic main body, which is optionally covered with one or more intermediate layers, **characterised in that** a coating applied to the implant to increase the tissue compatibility comprises a polysaccharide layer made of:
(a) chitosan and
(b) hyaluronic acid and/or hyaluronic acid derivatives.

2. The implant according to claim 1, **characterized in that** the polysaccharide layer contains chitosan in partial areas or partial layers.

3. The implant according to claim 2, **characterized in that** the polysaccharide layer comprises an adhesion-promoting layer made of chitosan.

4. The implant according to claim 3, **characterized in that** the adhesion-promoting layer is 0.1 to 50 µm, particularly 1 to 10 µm, thick.

5. The implant according to one of the preceding claims, **characterized in that** a component of the chitosan in the total weight of the polysaccharide layer is not more than 50 weight-percent.

6. The implant according to claim 1, **characterized in that** the hyaluronic acid and hyaluronic acid derivatives have an average molecular weight between 300,000 and 500,000 Dalton after sterilization of the implant.

7. A coating system according to claim 6, **characterized in that** the average molecular weight is between 380,000 and 420,000 Dalton.

8. The implant according to one of the preceding claims, **characterized in that** the polysaccharide layer has a composition such that the in vivo degradation of the polysaccharide layer is slowed from the outside in the direction of the main body of the implant.

9. The implant according to claim 8, **characterized in that** an internal area of the polysaccharide layer is not degradable in vivo, at least not completely, within two years.

10. The implant according to claim 9, **characterized in that** the internal area is 3 to 50 µm, particularly 5 to 20 µm, thick.

11. The implant according to claim 8, **characterized in that** an external area of the polysaccharide layer is degradable in vivo within 100 days.

12. The implant according to claim 11, **characterized in that** the external area is 10 to 250 µm, particularly 50 to 150 µm, thick.

13. The implant according to claim 8, **characterized in that** the polysaccharide layer comprises at least two partial layers having different degradation behaviours, the degradation behaviour within each partial layer being able to be fixed continuously changeably or constant over the partial layer.

14. The implant according to claim 13, **characterized in that** the polysaccharide layer comprises an internal partial layer which is degradable in vivo by not more than 20 weight-percent within 2 years.

15. The implant according to claim 4, **characterized in that** the internal partial layer is 3 to 50 µm, particularly 5 to 20 µm, thick.

16. The implant according to claim 13, **characterized in that** the polysaccharide layer comprises an external partial layer which is degradable in vivo by at least more than 50 weight-percent within 100 days.

17. The implant according to claim 16, **characterized in that** the external partial layer is 10 to 250 µm, particularly 50 to 150 µm, thick.

18. The implant according to one of the preceding claims, **characterized in that** a layer thickness of the polysaccharide layer is between 10-400 µm.

19. The implant according to claim 18, **characterized in that** the layer thickness is 50-120 µm.

20. The implant according to one of the preceding claims, **characterized in that** the hyaluronic acid, the hyaluronic acid derivatives, and the chitosan are components of the polysaccharide layer as individual substances, copolymers or block polymers made of hyaluronic acid, hyaluronic acid derivatives and chitosan, or in the form of mixtures of the above-mentioned individual substances and polymers.

21. The implant according to one of the preceding claims, **characterized in that** the polysaccharide layer is immobilized covalently or through physisorption on the implant.

22. The implant according to one of the preceding claims, **characterized in that** the implant is an endovascular implant, particularly a stent.

23. The implant according to one of the claims 1 to 21, **characterized in that** the implant is a stimulation electrode, which can be used with an implantable tissue stimulator, in particular with a pacemaker, a heart defibrillator, a bone stimulator or a neurostimulator.

## Revendications

1. Implant avec un corps de base métallique qui peut être éventuellement recouvert avec une ou plusieurs couches intermédiaires, **caractérisé en ce que**, pour augmenter la comptabilité cellulaire, on applique sur l'implant un revêtement composé d'une couche de polysaccaride de
(a) Chitosane et
(b) l'acide hyaluronique et/ou des dérivés d'acide hyaluronique

2. Implant selon la revendication 1, **caractérisé en ce que** la couche de polysaccharide comprend dans des zones partielles ou des couches partielles du chitosane.

3. Implant selon la revendication 2, **caractérisé en ce que** la couche de polysaccharide comprend une couche médiatrice d'adhésion de chitosane.

4. Implant selon la revendication 3, **caractérisé en ce que** la couche médiatrice d'adhésion a une épaisseur comprise entre 0,1 à 50 µm, notamment 1 à 10 µm.

5. Implant selon une des revendications précédentes, **caractérisé en ce qu'**un pourcentage du chitosane par rapport au poids total de la couche de polysaccharide n'est pas supérieure à 50% en poids.

6. Implant selon la revendication 1, **caractérisé en ce** les acides hyaluroniques et les dérivés hyaluroniques présentent après une stérilisation de l'implant, un poids moléculaire moyen compris entre 300.000 à 500.000 Dalton.

7. Système de revêtement selon la revendication 6, **caractérisé en ce que** le poids moléculaire moyen se situe entre 380.000 à 420.000 Dalton.

8. Implant selon une des revendications précédentes, **caractérisé en ce** la couche de polysaccharide est réalisée de manière à ralentir la dégradation in vivo de la couche de polysaccharide de l'extérieur en direction du corps de base de l'implant.

9. Implant selon la revendication 8, **caractérisé en ce qu'**une zone interne de la couche de polysaccharide ne peut pas être au moins pas complètement décomposée in vivo sous deux ans.

10. Implant selon la revendication 9, **caractérisé en ce** la zone interne a une épaisseur de 3 à 50 µm, notamment 5 à 20 µm.

11. Implant selon la revendication 8, **caractérisé en ce qu'**une zone extérieure de la couche de polysaccharide peut être décomposée in vivo sous 100 jours.

12. Implant selon la revendication 11, **caractérisé en ce** la zone extérieure a une épaisseur de 10 à 250 µm, notamment 50 à 150 µm.

13. Implant selon la revendication 8, **caractérisé en ce** la couche de polysaccharide comprend au moins deux couches partielles avec un comportement de dégradation différent; le comportement de dégradation pouvant être défini à l'intérieur de chaque couche partielle de façon continuellement variable ou constante sur la couche partielle.

14. Implant selon la revendication 13, **caractérisé en ce** la couche de polysaccharide comprend une couche partielle interne qui ne peut pas être décomposée in vivo de plus de 20% en poids en l'espace de 2 ans.

15. Implant selon la revendication 14, **caractérisé en ce** la couche partielle interne a une épaisseur de 3 à 50 µm, notamment 5 à 20 µm.

16. Implant selon la revendication 13, **caractérisé en ce** la couche de polysaccharide comprend une couche partielle extérieure, qui peut être décomposée in vivo d'une valeur au moins supérieure à 50% en poids en l'espace de 100 jours.

17. Implant selon la revendication 16, **caractérisé en ce** la couche partielle extérieure a une épaisseur de 10 à 250 µm, notamment 50 à 150 µm.

18. Implant selon une des revendications précédentes, **caractérisé en ce qu'**une épaisseur de couche de la couche de polysaccharide se situe entre 10-400 µm.

19. Implant selon la revendication 18, **caractérisé en ce** l'épaisseur de la couche s'élève à 50 -120 µm.

20. Implant selon une des revendications précédentes, **caractérisé en ce** les acides hyaluroniques, les dérivés d'acides hyaluroniques et le chitosans se présentent sous la forme de substances individuelles, co- ou polymères bloque d'acides hyaluronique, de dérivés d'acides hyaluroniques et de chitosane ou sous la forme de mélanges des substances individuelles mentionnées ci-dessus et de polymères faisant partie de la couche de polysaccharide.

21. Implant selon une des revendications précédentes, **caractérisé en ce** la couche de polysaccharide est immobilisée de façon covalente ou par physisorption sur l'implant.

22. Implant selon une des revendications précédentes, **caractérisé en ce** l'implant est un implant endovasculaire, notamment une endoprothèse vasculaire.

23. Implant selon une des revendications 1 à 21, **caractérisé en ce** l'implant est une électrode de stimulation pour être utilisée avec un stimulateur de tissu susceptible d'être implanté, notamment un pacemaker, un défibrillateur, un stimulateur osseux ou neurologique.
